# EUROPEAN PATENT APPLICATION

(11) **EP 3 385 271 A1**
(43) Date of publication of application: **10.10.2018**
(21) Application number: 17164701.9
(22) Date of filing: 04.04.2017
(51) Int. Cl.: C07H 1/08, C13K 13/00

(54) **INDUSTRIAL-SCALE D-MANNOSE EXTRACTION FROM D-MANNOSE BISULFITE ADDUCTS**

(71) Applicant: Borregaard AS, 1721 Sarpsborg (NO)
(72) Inventor: The designation of the inventor has not yet been filed
(74) Representative: Tostmann, Holger Carl

(57) **Abstract**

The present invention relates to a process for the selective isolation of highly purified, crystalline D-mannose from complex sugar mixtures, in particular from mixed wood sugars, more particularly from spent sulfite liquor (SSL). The process of the present invention is based on converting mannose into essentially pure mannose bisulfite adducts. Subsequent *oxidative* recovery of mannose from the mannose bisulfite adducts renders crystalline mannose in improved yields and purities.

## Description

The present invention relates to a process for the selective isolation of highly purified, crystalline D-mannose from complex sugar mixtures, in particular from mixed wood sugars, more particularly from spent sulfite liquor (SSL). The process of the present invention is based on converting mannose into essentially pure mannose bisulfite adducts. Subsequent *oxidative* recovery of mannose from mannose bisulfite adducts renders crystalline mannose in improved yields and purities.

### Background and Prior Art

D-mannose is a sugar, more specifically a hexose and the C-2 epimer of glucose. Mannose is important in human metabolism and is produced, in the human body, from glucose.

Highly pure mannose can be used in various applications of commercial relevance (for a review, see, for example: Hu, X.; Zhang, P.; Miao, M.; Zhang, T.; Jiang, B. Compr. Rev. Food Sci. Food Saf. 2016, 15, p. 773). First and foremost, D-mannose, in particular highly purified D-mannose, may be used as a dietary supplement or as a sweetener (lower calorie variant). Another closely related use for D-mannose involves the treatment of recurring urinal tract infection (UTI). It is believed that D-mannose can inhibit colonization/adhesion of certain bacterial strains in the body thus decreasing the risk of UTI. In the cosmetic industry, D-mannose is used both as moisturizer and as a component to improve skin feel.

Most plant varieties contain D-mannose and could be used, in principle, as a source for extracting the same. However, in nature, mannose is typically not available as a monomeric sugar. Rather, mannose is typically found as a component in various plant polymers including, but not limited to mannans and hemicellulose. A particularly interesting potential source for mannose is softwood as commonly used in the paper and cellulose-industry. Relatively large quantities of D-mannose are present in the form of carbohydrate polymers and copolymers in this particular raw material.

However, isolation of D-mannose from such complex mixtures of (lignocellulose) sugars is not straightforward. For example, spent sulfite liquor (SSL) as obtained after sulfite pulping operations may contain, in addition to D-mannose (polymers), a vast range of other water soluble polymers, lignosulfonates, extractives, cooking chemicals (salts) and other chemical entities, which are sometimes ill-defined. Owing to the complexity of these mixtures, isolation of any single component from wood sugar mixtures, in particular from SSL, has been historically difficult.

One method that is known, in principle, to remove mannose from complex sugar mixtures is to treat such a complex sugar mixture with a sulfite, resulting *in "mannose-bisulfite-adducts".* Although, in general, *formation* of an *"aldehyde-bisulfite adduct"* may be relatively straightforward, *regeneration* and *isolation* of the aldehyde is not, in particular not from complex mixtures. Typically, the aldehyde is regenerated by treatment of the aldehyde bisulfite adducts with an aqueous acid or a base, thus hydrolyzing the sulfite adduct into its separate components. The sulfite-ion must be efficiently removed from the reaction medium to ensure that the equilibrium reaction proceeds favorably towards the product. The hydrolysis step is typically followed by either distillation or extraction to separate the aldehyde from the residual sulfite salt.

The regeneration of the aldehyde from the bisulfite adduct can be particularly difficult when the target aldehyde is a sugar, in particular for the following reasons:
- treatment of sugars at high pH induces epimerization, and thus decreases the sugar purity;
- (complete) removal of sulfite from sugar is difficult and even traces of sulfite render mannose ill-suitable for certain applications.

US 3 677 818 describes the formation and isolation of D-mannose bisulfite adducts from a D-mannose-rich spent sulfite liquor (SSL) stream. US 3 677 818 discloses the treatment of SSL with Na-metabisulfite in a reaction medium consisting of water and an alcohol. After several days, a crystalline product forms and is separated from the reaction by filtration. The procedure yields a D-mannose bisulfite adduct in good yield, contaminated with lignosulfonate residues. US 3 677 818 further describes methods to regenerate D-mannose from D-mannose bisulfite adducts. A first method is based on treatment of the D-mannose bisulfite adduct with a base under semi-aqueous alkaline conditions, while a second method relies on treatment of the D-mannose bisulfite adduct with an acidic ion-exchange resin, followed by a basic ion-exchange resin.

US 5 994 593 describes the regeneration of an aldehyde bisulfite adduct by acidic hydrolysis, followed by distillation of the desired *volatile* aldehyde. Kjell et. al. (J. Org. Chem. 1999, 64, p. 5722) found that aldehyde bisulfite compounds may be cleaved/hydrolyzed using trimethylsilyl chloride (TMSCI) in aqueous media. However, this approach is not suitable for the industrial scale (high costs and waste-intensive).

Another approach to isolate D-mannose from various plant hydrolysates is by chromatographic separation. For example, US 6 773 512 or EP 1 468 121 disclose the multistep chromatographic isolation of D-mannose derived from Ca-SSL. Separation was achieved by pumping a solution containing D-mannose through several chromatographic columns connected in series.

### Summary of the Invention

In light of the prior art, in particular in light of the drawbacks outlined above, a method for the separation of mannose from a complex sugar mixture is needed, in particular from complex sugar mixtures made from biological materials, such as a *"spent sulfite liquor"* feed. More particular, a method is needed that leads to D-mannose in comparatively high yields, while minimizing or avoiding epimerization of D-mannose during the process of isolation/purification.

These objects and others are achieved by a process for the isolation of purified D-mannose, comprising at least steps (a), (d) and (e), or any combination of these with any of the other steps disclosed in the following:
(a) providing a feed of mixed sugars, which feed comprises D-mannose, including D-mannose in the form of polymers or copolymers; or
(a') providing a spent sulfite liquor (SSL) feed, which comprises D-mannose, including D-mannose in the form of polymers or copolymers;
(b) subjecting the spent sulfite liquor feed from (a') to a pretreatment step, which pretreatment comprises the separation, preferably by ultrafiltration, of at least a portion of the high molecular weight lignosulfonates present in the SSL feed, resulting in an aqueous phase, preferably an aqueous permeate, comprising D-mannose;
(c) concentrating the feed of mixed sugars of step (a) or of step (a') or of step (b) to a predetermined dry matter content;
(d) adding at least one sulfite to the mixture of step (a) or, if present, of step (a') or step (b) or step (c), resulting in a D-mannose-bisulfite adduct;
(e) regenerating/recovering D-mannose from the D-mannose-bisulfite adduct of step (d) by treating said D-mannose-bisulfite adduct, preferably having a dry matter content of 30 wt.-% to 80 wt.-%, with at least one oxidant and at least one base.

In embodiments of the present invention the oxidant is selected from, without being limited to: peroxides, organic peroxides, ozone, hypochlorite (and its derivatives), air, oxygen or any other readily available oxidant. In preferred embodiments, the oxidant employed in step (e) is or comprises H₂O₂.

Optionally, the following step (f) is conducted after step (e):
(f) crystallizing D-mannose from the mixture of D-mannose and sulfate of step (e) by first precipitating sulfate.

### Sugar / (Spent Sulfur Liquor) Feedstock

In principle, In accordance with the present invention, no restrictions exist in regard to the raw material used as the sugar mixture feed of step (a).

In accordance with the present invention, any material containing D-mannose in any form (e.g. as a monomer in mixture with other epimers, as polymers or as copolymers and/or as derivatives) can be used as the feedstock for the separation of highly purified D-mannose. Such mixtures may be, among others, plant (pre-)hydrolysates or glucose epimerization mixtures.

In case a lignocellulosic feed is used, in particular in step (a'), generally, before any of the components of lignocellulose-based raw products can be accessed, the lignocellulose needs to be broken down, particularly in a process commonly known as *"pulping".* The lignocellulose can be broken down by various processes that result in mixed lignocellulosic sugars. Such processes include, but are not limited to: hydrolysis by chemical or mechanical means, in particular high pressure, high temperature and/or chemical treatment. The resulting mixture may be a hydrolysis mixture or a pre-hydrolysis mixture.

A chemical process that is preferred to arrive at mixed lignocellulosic sugars is pulping *("cooking")* cellulose raw material with at least one sulfite.

During such a sulfite pulping process, in particular if conducted at a low pH, mannan is partly degraded and the resulting pulping liquors (*"spent sulfite liquor",* SSL) typically contain substantial amounts of D-mannose, usually in a mixture with a variety of other lignocellulosic sugars.

In embodiments of the present invention, the feedstock used in step (a) is or comprises a lignocellulosic material.

Preferably, step (a) is step (a'):
(a') providing a spent sulfite liquor (SSL) feed, which comprises D-mannose, including D-mannose in the form of polymers or copolymers.

In embodiments of the present invention, softwoods are a preferred source for isolating D-mannose. In accordance with the present invention, *"softwood"* is meant to be derived from gymnosperm trees (e.g. connifers), wherein gymnosperms have no flowers or fruits, and have unenclosed or "naked" seeds on the surface of scales or leaves. Gymnosperm seeds are often configured as cones. Particularly relevant examples of softwood are pine, spruce, cedar, fir and redwood.

In embodiments of the present invention, as a precursor to step (a'), a cellulose-based raw material is cooked with a sulfite, preferably a sodium, calcium, ammonium, or magnesium sulfite, under acidic, neutral or basic conditions.

This step *("sulfite cook")* dissolves most of the lignin present in cellulose as sulfonated lignin (lignosulfonate), typically together with parts of hemicellulose. The dissolved or liquid phase *("pulping liquor")* is the liquid spent sulfur liquor (SSL) phase to be used for D-mannose extraction in accordance with the preferred embodiments of the present invention.

In preferred embodiments of the present invention, D-mannose is isolated from sulfite liquor derived from the digestion of softwood by using a sulfite pulping step using calcium sulfite *("Ca base").*

### Pretreatment Step

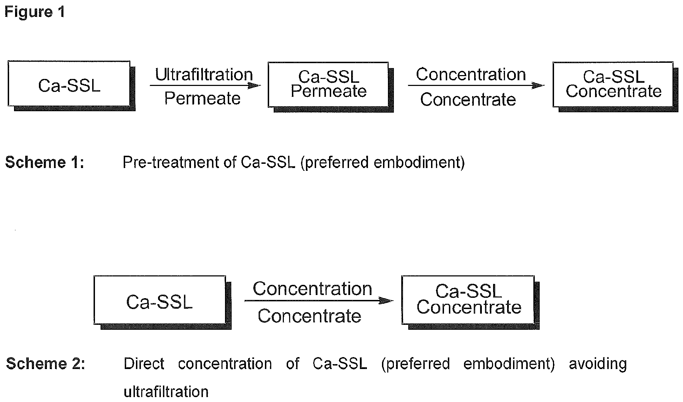

In embodiments of the present invention, the sulfite spent liquor stream from (a') is subjected to a pretreatment step, which comprises a separation, preferably by ultrafiltration, wherein at least a portion of the high molecular weight lignosulfonates (LS) as present in the SSL feed, and/or other components present in the SSL, are retained by the separation means, in particular by filter means, and are separated from an aqueous phase, which is preferably present as a permeate, comprising D-mannose (see Scheme 1, **Figure 1**).

Other preferred pretreatment steps are size exclusion chromatography (SEC) and ion-exchange chromatography.

Specifically, in these embodiments, step (b) follows step (a'):
(b) subjecting the spent sulfite liquor (SSL) feed from (a') to a pretreatment step, which pretreatment comprises the separation, preferably by ultrafiltration, of at least a portion of high molecular weight lignosulfonates present in the SSL feed, resulting in an aqueous phase, preferably an aqueous permeate, comprising D-mannose.

It has been found that pretreating the SSL in this manner, in particular by ultrafiltration, facilitates the isolation of D-mannose in the overall process.

In embodiments of the invention, the *"high molecular weight"* lignosulfonates in step (b) have a molecular weight cut-off in the range of 2kDa to 100 kDa, preferably 10 kDa to 100 kDa.

While pre-treatment involving ultrafiltration is a preferred embodiment, it is also within the scope of the present invention to not implement this additional pretreatment step and to directly concentrate the spent sulfur liquor from step (a') to a predetermined *dry matter content* [in step (c)] (see Scheme 2, **Figure 1**).

### Concentration Step

In embodiments of the invention, the feed of mixed sugars from (a) or the spent sulfur liquor feed (a') or the mixture from step (b) is concentrated to a predetermined dry matter content

In embodiments of the invention, the predetermined dry matter content achieved in step (c) is 20% to 70% of solid matter in % weight relative to the overall weight, preferably 35% to 55%, further preferably 40% to 50%.

In this step, a solvent, in particular water, is at least partly removed from the mixture/solution of step (a) or step (a')/(b).

In embodiments of the present invention, the solvent, preferably water, is at least partly removed by means of applying heat and/or by means of applying a reduced pressure vis-à-vis atmospheric pressure, preferably by means of applying a vacuum, in order to obtain a liquid concentrate.

In accordance with the present invention, no limitations exist how the solvent, in particular water, is removed from the solvent/mixture. Removal (stripping) by applying a vacuum and or applying heat is/are preferred.

### Formation of D-Mannose Bisulfite Adducts

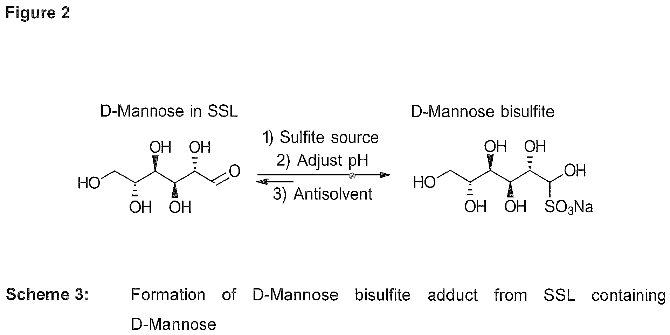

The mixed sugar/SSL feed from step (a) or step (a')/(b), obtained with or without pre-treatment and with or without further concentration [step (c)], is subsequently brought into contact with a source of sulfite, in an aqueous solution (see Scheme 3, **Figure 2**).

Specifically, said step is step (d) and comprises:
(d) adding at least one sulfite to the mixture of step (a) or, if present, of step (a'), step (b) or step (c), resulting in a D-mannose-bisulfite adduct;

This step is part of the overall process, based on the realization that aldehydes, in particular sugars, react with a sulfite source to form what may be characterized as an *"aldehyde bisulfite adduct".* These aldehyde derivatives are preferably crystalline materials, facilitating their separation and purification. In accordance with the present invention, the adduct of interest specifically is a *"D-mannose bisulfite adduct".*

In embodiments of the present invention, the sulfite added may be based on essentially any sulfite-source, including but not limited to, preferably, the Na⁺, Ca²⁺, NH₄⁺, K⁺-salts of either sulfite, bisulfite and/or metabisulfite, including SO₂ as a liquid or a gas, or any mixture thereof.

Preferably, an excess of said sulfite is added, but stoichiometric or sub-stoichiometric amounts of sulfite can also be used.

In preferred embodiments of the present invention, at least one anti-solvent is added to the reaction mixture, after the step of adding of at least one sulfite. Preferably, said anti-solvent is a short chain alcohol, such as methanol, ethanol or iso-propanol. Ethanol is particularly preferred as anti-solvent. It is also within the scope to not use any anti-solvent at this stage, i.e. the addition of an anti-solvent is optional.

### Filtration and Working up of the Bisulfite-Adduct

After the adduct formation with sulfite and, preferably, after addition of an anti-solvent, the reaction mixture is left to crystallize at a predefined temperature and for a predetermined time period.

After crystallization, the solid is filtered off, using any method for filtering known to the skilled person, for example, but not limited to: centrifugation, pressure filtration or vacuum filtration, resulting in a wet filter cake.

In embodiments of the present invention, the wet filter cake is then washed with a solvent. In a preferred embodiment, said solvent comprises at least water and ethanol. A particularly preferred solvent is a solution of 25-50 vol% water in ethanol, relative to the overall volume of the solvent.

As a result of the bisulfite-adduct forming step, and in accordance with the present invention as described above, the resulting D-mannose-containing mixture is essentially free of other sugars.

In embodiments of the invention, the amount of sugars *other than mannose,* after step (d) and before step (e) is less than 5% by weight, relative to the overall weight of the mixture, preferably less than 1%.

After the washing step, essentially all of the mother liquor is removed from the solid phase. In accordance with the present invention, there is no limitation in regard to how the mother liquor is removed. Methods of mother liquor removal may include, but are not limited to: drying by heat, drying by reduced pressure, centrifuging, etc.

In embodiments of the invention, the D-mannose bisulfite product resulting from said step (d) preferably has a dry matter content of 30 to 80 wt.-%, preferably 40 to 70% (based on the overall weight) and/or a D-mannose content of 50 to 70 wt.-%, preferably 55% to 60 wt% (based on the overall dry mass).

### Regeneration of Free D-mannose by Using an Oxidant

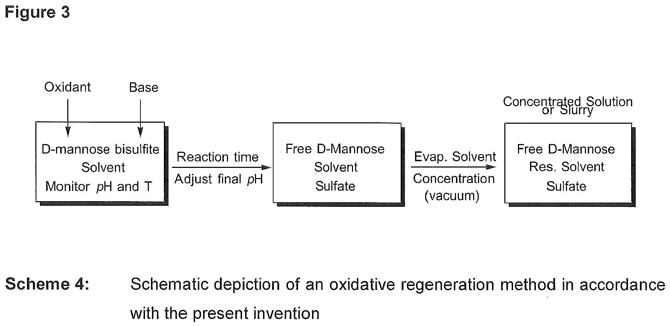

The D-mannose bisulfite adduct from the filtration and working-up steps as outlined above is now treated with at least one oxidant, together with at least one base (see Scheme 4, **Figure 3**).

Specifically, and in accordance with the present invention, the overall process comprises the following step (e):
(e) regenerating (recovering) D-mannose from the D-mannose-bisulfite adduct of step (d) by treating said D-mannose-bisulfite adduct, preferably having a dry matter content of 30 wt.-% to 80 wt.-%, with at least one oxidant and at least one base.

In embodiments of the present invention the oxidant is selected from, without being limited to: peroxides, organic peroxides, ozone, hypochlorite (and its derivatives), air, oxygen or any other readily available oxidant. A preferred oxidant is H₂O₂.

In embodiments of the invention, the at least one base is chosen from, without being limited to: alkali/ earth alkaline metal hydroxides and carbonates as well as organic bases. NaOH or KOH in solid form are particularly preferred.

In embodiments of the invention, the pH value does not exceed 7 during the entire oxidation step (e). The oxidant is preferably added slowly to an aqueous slurry of D-mannose bisulfite adduct, with simultaneous addition of a base.

During addition of the oxidant and base, the pH preferably is controlled to be in the range of from 1 to 5. Preferably, the addition rate is adjusted to maintain pH in the range between 3 and 4.

Overall, the reaction is exothermic (neutralization of acid and heat of oxidation) and is preferably performed between 0°C and. 80°C, further preferably in the range of from 10°C to 40°C.

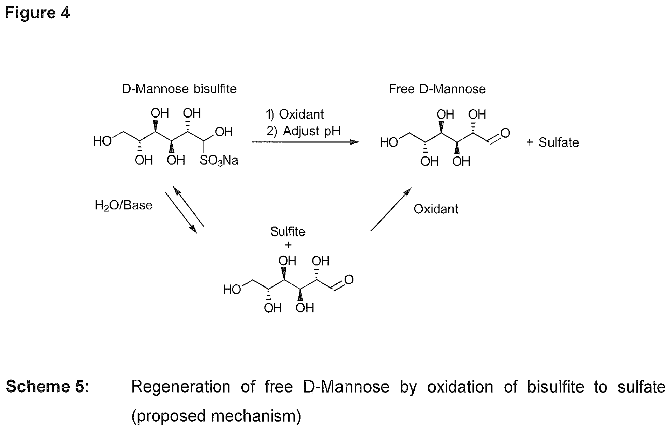

Without wishing to be bound by theory, it is believed that the D-mannose bisulfite adduct is not (directly) oxidized in this step. Rather, it is believed that a fraction of the D-mannose bisulfite adduct hydrolyzes into D-mannose and free *"sulfite".* In a subsequent step, the free sulfite is rapidly converted to a *"sulfate"* (see Scheme 5, **Figure 4**), hence driving the reaction forward.

Using an oxidant in the present oxidation step (e) has, among others, the following advantages vis-à-vis other conceivable regeneration methods, in particular (bi)carbonate treatment (alkaline conditions), as described in the art:
- Regeneration using an oxidant in accordance with the present invention is performed under aqueous *acidic* conditions, avoiding base catalyzed epimerization of D-mannose occurring in alkaline solution (for example in the presence of a carbonate). Specifically, using oxidation for regeneration is less sensitive to variations in the reaction time as opposed to the regeneration with a carbonate base. For example, using the base catalyzed hydrolysis as described in US 3 677 818, the reaction time must be kept comparatively short to avoid epimerization.
- The oxidant-based regeneration (recovery) method in accordance with the present invention generates non-toxic sodium sulfate as waste instead of sodium sulfite, which is of particular relevance for food/cosmetics applications of D-mannose.
- The oxidant-based regeneration (recovery) method according to the present invention accepts poor starting D-mannose bisulfite adducts, while providing crystalline D-mannose in comparatively high yield and purity in the end.

### Salt Precipitation (Removal) and Crystallization of Mannose

When the oxidation reaction of step (e) is complete, typically, significant quantities of sulfate have formed (typically Na₂SO₄, with a lesser amount of CaSO₄, in case NaOH was used as the base and a Ca-based SSL was used as the feed, the corresponding salts will form, if another counter ion is present in the base and/or the SSL feed).

In embodiments of the present invention, the resulting salt is removed from the solution of step (e) in a working-up step that is conducted after the oxidation step, preferably by a precipitation/filtration procedure.

Therefore, in preferred embodiments the following step (f) is conducted after step (e):
(f) crystallizing D-mannose from the mixture of D-mannose and sulfate of step (e) by first precipitating sulfate.

In preferred embodiments of the present invention, between 50 wt.-% and 100 wt.-% of the total mass of water is distilled off the slurry of salt and D-mannose at low pressure, preferably by means of evaporation under slightly elevated temperature (for example at 60 to 100 mbar, and in a temperature range of from 60°C to 70°C). The resulting slurry is enriched in D-mannose and has a predetermined low water content. Other methods of removing salt from a solution may also be used, including, but not limited to: osmosis, electro-dialysis or ion-exchange.

In embodiments of the present invention, at least one solvent is added to the slurry resulting from the water removal step, the previous step, preferably ethanol, further preferably ethanol mixed with water. Other solvents, in particular lower alcohols, are equally suited for this task.

In preferred embodiments, the solvent addition step precipitates all or at least a significant part of the salts, in particular the sulfates, as a solid phase. This process is preferably conducted in a temperature range of from 40°C to 60°C, so that all or most of the D-mannose remains in the solution.

The final water concentration of the solvent/D-mannose solution is preferably in the range of 2 wt.-% to 15 wt.-%, preferably from 5 wt.-% to 10 wt.-% water relative to the weight of the overall solution. At this water content, improved crystallization results (see below) are achieved.

However, it is within the scope of the present invention to use both higher and lower quantities of water, as long as crystallization of D-mannose occurs. The concentration of D-mannose in the salt reduced solution is preferably adjusted to be between 5% to 20%, with 12% to 18% being particularly preferred.

The desalinated solution containing the dissolved D-mannose is then filtered off from the sulfate using any standard filtration technique known to the skilled person (decantation, vacuum filtration, pressure filtration or centrifugation), resulting in a solution of D-mannose in a solvent, preferably in alcohol (see above).

After the salt-removal step, the resulting filtrate is crystallized, preferably directly crystallized by seeding. In preferred embodiments, the solution from the salt-removal (or reduction) step is directly fed into a crystallization vessel, and seeded with pure D-mannose. D-mannose is then allowed to crystallize by slowly lowering the temperature from 50°C to 10°C during 24 to 48 hours.

The final crystalline product is then washed, preferably washed with a mixture of EtOH and water, further preferably with a mixture containing between 5 to 10 vol% water in EtOH.

The washed product is then preferably dried under vacuum (preferably at 20-50°C) to yield crystalline D-mannose in approximately 98% to 100% purity (IC analysis; see Examples).

The combination of oxidative regeneration and precipitation of sulfate(s) has the advantage that high purity (98-100%) D-mannose can be readily obtained directly from the filtrate, after salt-precipitation, without any further processing steps.

In addition, owing to the fact that the regeneration has been conducted with an oxidant, in particular with H₂O₂, the final product is essentially free of sulfites. Noticeable presence of sulfites has a negative effect on smell and taste of the crystalline D-Mannose, even with only traces of sulfite present.

### Brief Description of the Figures

- **Figure 1**: shows an exemplary embodiment of the present invention, in which a Ca SSL feed is pretreated by ultrafiltration and then concentrated (Scheme 1) as well as an alternative embodiment, in which the feed is directly concentrated.
- **Figure 2**: shows an exemplary embodiment of the present invention, in which D-mannose in SSL is converted into a D-mannose bisulfite adduct.
- **Figure 3**: shows an exemplary embodiment of the present invention, in which the D-mannose bisulfite adduct is converted into *"free"* mannose by an oxidative treatment.
- **Figure 4**: depicts the reaction mechanism believed to represent the conversion of D-mannose bisulfite to "*free*" mannose.

### Detailed Description of Embodiments of the Present Invention

### Raw materials

In respect to the raw material to be used as feedstock for D-mannose extraction, no limitations exist, in principle, as long as the material contains D-mannose in some form (e.g. as polymers or as co-polymers), for instance as mannan, xylomannan, galactomannan, glucomannan or galactoglucomannan.

Mannan is abundant in nature, and is present, for example, in the endosperm of the monocotyledonous (e.g. Arecaceae family), such as ivory nuts (Phytelephas sp.), dates (Phoenix dactylifera), oil palm kernels (Elaeis guineensis) and coconut (Cocos nucifera). Mannan is also found in the cell walls of several green algae belonging to the Codiaceae and Dasycladaceae families. It is also distributed within some species from Apiaceae, Rubiaceae (Coffea sp.) and Asteraceae. Furthermore, mannan is found to be a major constituent of Mycobacteriae and yeast cell walls. Xylomannan is found in red seaweed (Nothogenia fastigiata).

Galactomannan is mainly found in leguminous seeds such as Fenugreek (Trigonella foenum-graecum), Guar (Cyamopsis tetragonoloba), Tara (Caesalpinia spinosa) and Carob (Ceratonia siliqua). Galactomannan is also a component of the cell wall of the mold Aspergillus.

Glucomannan is found in the roots of the konjac plant (Amorphophallus konjac). It is also a hemicellulose that is present in large amounts in the wood of conifers and in smaller amounts in the wood of dicotyledons.

Galactoglucomannan are prominent components of coniferous woods (almost 20% of dry matter from softwoods are hemicellulosic galactoglucomannan). These are also found in other wood species as well as in the primary cell walls of other plants, fruits, and seeds, such as flax, tobacco plants, or kiwifruit.

A suitable feed of mixed sugars may be or comprise a hydrolysate or a prehydrolysate in particular of biological material with mannose containing polymers such as mannan, xylomanna, glucomannan, galactomannan or galactoglucomannan, or a glucose epimerization mixture.

The raw material for the feed is selected from annual plants, corms, in particular konjac, yeast and fungi cell walls, in particular Saccharomyces cerevisiae, agricultural residues or food residues, in particular copra meal, palm kernel meal, spent coffee grounds or orange peel, or wood, in particular softwood.

Preferred raw materials that are suited for the process of the present invention are energy crops, annual plants, agricultural residues and wood. One advantage of such materials is that not only that D-mannose may be extracted, but that the cellulose pulp can be further processed to valuable products, for example by way of converting the lignocellulosic biomass in a biorefinery process (biomass conversion).

### Sulfite Pulping

Unlike the sodium based Kraft process that is performed at a pH of the fresh cooking liquor of about 13, "sulfite cooking" is characterized in that it covers the whole pH range. The pH may range from < 1 (using sulfur dioxide solutions in water) to > 13 (using sulfur dioxide or sodium sulfite or sodium bisulfite together with sodium hydroxide).

Sulfite cooking may be divided into four main groups: acid, acid bisulfite, weak alkaline and alkaline sulfite pulping.

In embodiments of the present invention and prior to step (a'), the lignocellulosic biomass is cooked with a sulfite, preferably a sodium, calcium, ammonium or magnesium sulfite, further preferably calcium sulfite under acidic, neutral or basic conditions.

This sulfite cook dissolves most of the lignin as sulfonated lignin (lignosulfonate) together with parts of the hemicellulose, if present. This dissolved or liquid phase (pulping liquor) is the liquid SSL phase. The cellulose is left almost intact in the pulp, together with parts of the hemicellulose.

### Separation of Pulp and SSL

Prior to step (a'), the pulp (solid phase; cellulose and hemicellulose) is preferably separated from the spent sulfite liquor (liquid SSL phase; SSL, sulfonated lignin and hemicellulose) by any separation method known to the person skilled in the art; in particular pressing, filtration, sedimentation or centrifugation.

### EXAMPLES

### Example 1:

### Preparation of a Spent Sulfite Liquor

Spent Sulfite Liquor (SSL) was derived from the digestion of softwood chips with calcium sulfite, in a pulping step ("Ca-based" SSL).

The sulfite spent liquor stream was then subjected to a pretreatment step of ultrafiltration (UF, 2-100KDa MWCO), in which the high molecular weight lignosulfonates (LS), and any other substances retained by the membrane were separated from the aqueous permeate containing the D-mannose (see Scheme 1 in **Figure 1**).

Some of the water was then stripped off the D-mannose rich Ca-SSL-permeate solution under vacuum, in order to obtain a Ca-SSL concentrate. This brown Ca-SSL concentrate was then used to prepare the mannose-sulfite adduct.

### Example 2:

### Preparation and Working-up of the Mannose-Sulfite Adduct

The aqueous Ca-SSL concentrate obtained with pre-treatment by ultrafiltration was contacted with an excess of sulfite, in an aqueous solution (see Scheme 3, **Figure 2**).

The resulting solution was then stirred and the *p*H was adjusted to pH ≈ 4. A short chain alcohol was then added to the reaction as an anti-solvent, while maintaining a reaction temperature of 60°C. The resulting solution was then held at 60°C for 30 hours before being cooled to 20°C for a time period of 5 to 7 hours.

The mixture was left to crystallize under a set temperature program for 18 to 24 hours, in which time period a thick beige/white slurry of solids developed. The solid was then filtered off using vacuum filtration to provide a wet filter cake. The so-obtained D-mannose-bisulfite adduct was essentially free from other sugars. The filter cake was then washed with a solvent consisting of water and ethanol (25-50 vol% water in ethanol). After the washing procedure, the essentially colorless white solid was centrifuged/pressure dried to remove most of the mother liquor from the wet filter cake. The wet D-mannose bisulfite product typically had a dry matter content of 30 to 80 wt.-% and a D-mannose content of 50 to 60 wt.-% (based on dry mass).

### Example 3:

### Oxidation of the Mannose-Sulfite Adduct (Laboratory Scale)

1.0 kg of mannose-bisulfite adduct (equivalent to 500 g D-mannose) were added into a 5 L reactor, followed by the addition of 2.0 L of water. The slurry was agitated using a mechanical overhead stirrer and the reactor was fitted with a thermometer, pH-probe and a 500 mL addition funnel. The reaction temperature (jacket) controlled using a temperature controlled thermostat.

330 to 340 mL of H₂O₂ (35% aq.) were added to the slurry of D-mannose bisulfite adduct, via the addition funnel over the course of 15-20 minutes. 50% aq. NaOH was simultaneously added to the solution in such fashion that the temperature of the reaction did not exceed 40 to 45°C, while the pH was kept <4,5. In total, 250 to 260 g of 50%, aq. NaOH was needed for the transformation. The NaOH addition time was typically 20 to 40 minutes. After addition of approximately 4/5 of the volume (or mass) of 50% aq. NaOH, the mixture had become an essentially clear solution. At this point, the pH also rose slowly from pH 1,5 to 2,5 to pH 3 to 4. Addition of NaOH was stopped at pH=5. After complete addition of both H₂O₂ and 50% aq. NaOH, the solution was cooled to 20 to 25°C (at pH=5) and left to stir at this temperature for 1 hour. At this time a sample was collected and filtered through a 0,45 µm syringe filter. The residual H₂O₂ content at this point was <250ppm, as determined by peroxide sticks (Quantostix).

Two drops of the filtrate were diluted with D₂O and subjected to ¹H-NMR spectroscopy to determine if all of the D-mannose bisulfite adduct had reacted. D-mannose bisulfite adduct has a characteristic signal at∼ 4,14 ppm (d, *J* = 9,5 Hz), readily traceable in the ¹H-NMR-spectrum

### Optional de-colorization step

In an optional step, 5g of charcoal (1 wt% based on dry D-Mannose in D-mannose bisulfite adduct) is added to the oxidized solution obtained after addition of H₂O₂ and NaOH. The resulting slurry was stirred for 1 hour at pH 4,5-5 (20-23°C). After this time, the charcoal was filtered off under vacuum (fiber glass filter) to give a charcoal free decolorized solution. This operation has only a limited effect on the sugar content and sugar distribution in the solution.

The lightly yellow solution (before or after de-colorization with charcoal) was filtered and water was evaporated off (rotary evaporator, 60°C, water-suction) until a syrup like residue containing approx. 20-30 wt% water obtained (determined by Karl Fischer titration). This step provided a syrup/slurry containing large amounts of Na₂SO₄ and D-mannose.

### Example 4:

### Salt Precipitation

500 wt.-% EtOH (with respect to the total amount of D-mannose in the starting adduct) was added to the syrup containing D-mannose from Example 3. The slurry was heated to 50°C and maintained at this temperature for 1 hour to dissolve the sugars and precipitate the Na₂SO₄ (under mechanical-overhead stirring). The solids were then filtered off at 40 to 50°C (vacuum filtration, blue-ribbon filter paper) and the filter cake was washed with 100 wt% EtOH (with respect to the total amount of D-mannose in the starting adduct, EtOH/H₂O, 95:5 by wt. at 20°C) to provide a combined filtrate. The water content (Karl Fischer titration) was determined to be 7 to 10 wt.-% in the filtrate. On the basis of sugar analysis (ion chromatography), conversion/recovery of D-mannose from the D-mannose bisulfite adduct was found to be within the range of 95 to 100% (i.e. quantitative)

### Example 5:

### Crystallization

The warm filtrate obtained in example 4 (∼45°C) was seeded at 45°C with crystalline D-mannose and allowed to cool down slowly (∼0,8°C/h) from ∼45 °C to 10°C. Crystallization was allowed to proceed for 48 hours before the solid mass of product was filtered of at 10°C. The resulting filter cake was washed with a solution comprised of 95:5 EtOH/Water (by volume) cooled to 5°C to give a white crystalline solid. The solid was dried under vacuum at 45°C to provide crystalline D-mannose in 50 to 55% yield based on D-mannose found in the starting D-mannose bisulfite adduct. The dry product contained <0.15 wt.-% EtOH and <0.10 wt.-% residual water. The purity of the solid was ∼98-99% by ion chromatography and no other sugars than D-mannose could be detected (by ion chromatography). The ash content was typically <0.25 wt.-%.

## Claims

1. Process for the isolation of purified D-mannose, comprising at least the following steps:
(a) providing a feed of mixed sugars, which feed comprises D-mannose, including D-mannose in the form of polymers or copolymers; or
(d) adding at least one sulfite to the mixture of step (a) or, if present, to the mixture of step (a') or step (b) or step(c), resulting in a D-mannose-bisulfite adduct;
(e) regenerating D-mannose from the D-mannose-bisulfite adduct of step (d) by treating said D-mannose-bisulfite adduct, preferably having a dry matter content of 30 wt.-% to 80 wt.-%, with at least one oxidant and at least one base.

2. Process according to claim 1, comprising step (a') instead of step (a):
(a') providing a spent sulfite liquor (SSL) feed, which comprises D-mannose, including D-mannose in the form of polymers or copolymers;

3. Process according to claim 2, additionally comprising the following step:
(b) subjecting the spent sulfite liquor feed from (a') to a pretreatment step, which pretreatment comprises the separation, preferably by ultrafiltration, of at least a portion of the high molecular weight lignosulfonates present in the SSL feed, resulting in an aqueous phase, preferably an aqueous permeate, comprising D-mannose, preferably wherein the "high molecular weight" lignosulfonates have a molecular weight cut-off in the range of 2 kDa to 100 kDa, further preferably 10 kDa to 100 kDa.

4. Process according to any of the preceding claims, additionally comprising the following step, after step (a) or (a'), or after step (b):
(c) concentrating the feed of mixed sugars from step (a) or from step (a') or from step (b) to a predetermined dry matter content.

5. Process according to any of the preceding claims, additionally comprising the following step, after step (e):
(f) crystallizing D-mannose from the mixture of D-mannose and sulfate of step (e) by first precipitating sulfate.

6. Process according to any of the preceding claims, wherein the feed of mixed sugars is or comprises a hydrolysate or a prehydrolysate of biological material with mannose containing polymers such as mannan, xylomannan, glucomannan, galactomannan or galactoglucomannan, or a glucose epimerization mixture.

7. Process according to claim 6, wherein the raw material for the feed is selected from annual plants, corms, in particular konjac, yeast and fungi cell walls, in particular Saccharomyces cerevisiae, agricultural residues or food residues, in particular copra meal, palm kernel meal, spent coffee grounds or orange peel, or wood, in particular softwood.

8. Process according to any of claims 2 to 7, wherein, prior to step (a'), a lignocellulose-based raw material is cooked with a sulfite, preferably a sodium, calcium, ammonium, sodium or magnesium sulfite under acidic, neutral or basic conditions.

9. Process according to any one of the preceding claims, wherein the sulfite added in step (d) is selected from the Na⁺, Ca²⁺, NH₄⁺, K⁺-salts of either sulfite, bisulfite and/or metabisulfite, including SO₂ as a liquid or a gas, or any combination thereof.

10. Process according to any one of the preceding claims, wherein at least one solvent is added to the reaction mixture of (d).

11. Process according to any one of the preceding claims, wherein the D-mannose bisulfite product resulting from step (d) has a dry matter content of 30 to 70 wt.-% and/or a D-mannose content of 50 to 60 wt.-%, based on the overall dry mass.

12. Process according to any one of the preceding claims, wherein the D-mannose bisulfite product resulting from step (d) preferably has a dry matter content of 30 to 80 wt.-%, preferably 40 to 70% (based on the overall weight) and/or a D-mannose content of 50 to 70 wt.-%, preferably 55% to 60 wt% (based on the overall dry mass).

13. Process according to any one of the preceding claims, wherein the oxidant of step (e) is selected from peroxides, organic peroxides, ozone, hypochlorite (and its derivatives), air, oxygen, or any combination thereof, wherein H₂O₂ is preferred as oxidant.

14. Process according to any one of the preceding claims, wherein, in step (e), the pH at no time exceeds the value of "7", preferably wherein during the addition of the oxidant and base, the pH preferably is controlled to be in the range of from 1 to 5.
